Europäisches Patentamt

European Patent Office

Office européen des brevets

(19)

(11) Numéro de publication: **0 080 388**
**B1**

(12)

# FASCICULE DE BREVET EUROPEEN

(45) Date de publication du fascicule du brevet:
04.09.85

(51) Int. Cl.⁴: **C 07 C 139/04, C 07 C 91/04**

(21) Numéro de dépôt: 82401925.1

(22) Date de dépôt: 20.10.82

(54) Procédé pour préparer en continu des photosulfonates d'alcanolamine et/ou d'alcoylamine à partir d'esters gras ou de paraffines de pétrole et photosulfonates d'alcanolamine et/ou d'alcoylamine ainsi obtenus.

(30) Priorité: 06.11.81 FR 8120789

(43) Date de publication de la demande:
01.06.83 Bulletin 83/22

(45) Mention de la délivrance du brevet:
04.09.85 Bulletin 85/36

(84) Etats contractants désignés:
BE DE GB IT NL

(56) Documents cités:
DE - A - 1 418 031

(73) Titulaire: DUMAS et INCHAUSPE, Zone Industrielle Avenue d'Ossau, F-64121 Serres Castet (FR)

(72) Inventeur: Naon, Hubert, 9 rue du Comte Saint Cricq, F-64000 Pau (FR)

(74) Mandataire: Lepeudry-Gautherat, Thérèse et al, CABINET ARMENGAUD JEUNE CASANOVA et LEPEUDRY 23 boulevard de Strasbourg, F-75010 Paris (FR)

# Description

La présente invention concerne un procédé pour préparer en continu des photosulfonates d'alcanolamine et/ou d'alcoylamine résultant de la photosulfonation d'esters gras ou bien de paraffines de pétrole.

Par esters gras, il faut entendre les esters méthyliques saturés d'origine naturelle, c'est-à-dire obtenus à partir d'huiles végétales telles que l'huile de palme, l'huile de soja ou l'huile de colza, ou à partir de graisses animales telles que le suif. Ces esters gras ont des chaînes carbonées droites longues comprenant de 12 (ester de l'acide laurique) à 18 (ester de l'acide stéarique) atomes de carbone. Par paraffines de pétrole, il faut entendre des alcanes à chaîne droite non ramifiée dont la chaîne comprend 12 à 18 atomes de carbone, ainsi que leur mélange.

La photosulfonation des paraffines est bien connue et a donné lieu à de nombreux brevets. La photosulfonation des esters gras est notamment décrite dans la demande de brevet français N° 80-00521. La présente invention ne porte pas directement sur la réaction de photosulfonation elle-même, mais sur les étapes d'extraction et de neutralisation qui suivent l'étape de photosulfonation.

Lorsque l'on soumet, en effet, des esters gras ou des paraffines de pétrole à une sulfonation par un mélange d'anhydride sulfureux et d'oxygène sous irradiation, on constate que la sulfonation ne peut pas être menée jusqu'au bout, et que seule une fraction d'ester ou de paraffine a été photosulfonée. Pour rendre le procédé de photosulfonation industriellement rentable, il s'avère donc nécessaire de recycler vers l'étape de photosulfonation l'ester ou la paraffine n'ayant pas réagi, et pour cela, il faut trouver un agent d'extraction pour le composé sulfoné.

Dans le cas de la photosulfonation des paraffines c'est l'eau ou le méthanol, ou un mélange en quelconque proportion d'eau et de méthanol qui est utilisé (brevet français N° 1547452). Dans le cas de la photosulfonation des esters gras, le problème est compliqué par le fait que le méthanol est miscible aux esters gras et que l'eau les hydrolyse. On ne peut donc utiliser ni l'un ni l'autre isolément et la demande de brevet français N° 80-00521 définit les proportions étroites d'un mélange de méthanol légèrement aqueux par lequel on arrive à extraire les esters sulfonés, c'est-à-dire à séparer les esters insulfonés sans les hydrolyser.

Dans l'un et l'autre cas, les produits photosulfonés sont extraits à l'état d'acides sulfoniques et ces acides sont ensuite neutralisés à la soude, généralement pour donner les sulfonates de sodium utilisés dans les lessives du commerce.

Aussi, un des buts de la présente invention est-il de fournir un procédé permettant, simultanément, d'extraire les produits insulfonés et de neutraliser les produits sulfonés.

Un autre but de l'invention est un procédé de ce type qui peut être exploité de façon continue.

Ces buts ainsi que d'autres qui apparaîtront par la suite sont atteints, de façon surprenante, par le procédé selon la présente invention, qui comprend les étapes suivantes:

a) on soumet le produit de départ constitué par un ester gras de méthyle ou une paraffine de pétrole tel que défini ci-dessus, à une photosulfonation en milieu anhydre par un mélange d'anhydride sulfureux et d'oxygène sous irradiation ultra-violette;

b) on dégaze le produit issu de l'étape a de l'anhydride sulfureux qu'il contient;

c) on ajoute au produit dégazé obtenu à l'issue de l'étape b une quantité d'alcanolamine et/ou d'alcoylamine supérieure à celle qui est strictement nécessaire pour neutraliser les acides photosulfoniques contenus, de façon à décanter le mélange ainsi obtenu en deux phases, une phase légère dite raffinat et une phase lourde dite extrait;

d) on recycle vers l'étape a le raffinat qui renferme du produit de départ insulfoné;

e) on recueille l'extrait c qui renferme les acides sulfoniques.

De préférence, on ajoute à l'étape c une quantité d'alcanolamine et/ou d'alcoylamine suffisante pour élever le pH au moins jusqu'à 8.

Avantageusement, on réalise l'étape c à une température comprise entre 40 et 60°C.

De préférence, l'alcanolamine est choisie dans le groupe constitué par la monoéthanolamine, la diéthanolamine, la triéthanolamine et l'alcoylamine parmi la mono-isopropylamine, la diisopropylamine, la triisopropylamine ou leurs mélanges.

Selon un mode de réalisation préféré de l'invention, les esters gras de méthyle qui constituent le produit de départ sont obtenus par la méthylation des huiles et des graisses naturelles d'origine animale ou végétale, suivie d'une extraction ou d'une hydrogénation des composés insaturés.

Selon un autre mode de réalisation préféré, les paraffines de pétrole qui constituent le produit de départ ont des chaînes carbonées droites comportant 12 à 18 atomes de carbone, ou leur mélange.

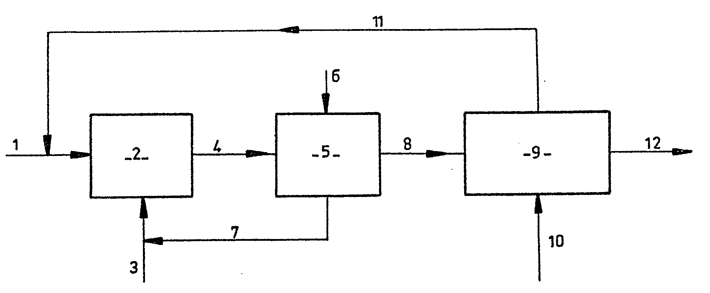

La description qui va suivre et qui ne présente aucun caractère limitatif doit être lue en regard de la figure unique annexée qui présente un synoptique du procédé selon la présente invention.

Ainsi qu'on peut le voir sur cette figure, les esters gras de méthyle ou les paraffines de pétrole qui constituent le produit de départ (1) sont introduits dans un réacteur de photosulfonation (2). Le mélange sulfonant (3) constitué par de l'anhydride sulfureux et de l'oxygène est introduit dans ce réacteur (2) où il est dispersé dans le milieu réactionnel par tout moyen d'injection gaz/liquide approprié. Ce réacteur (2) est soumis à une irradiation ultra-violette selon les modalités connues.

On soutire en continu du réacteur (2) le produit de sulfonation (4) obtenu à l'issue de l'étape a de photosulfonation ci-dessus, et on dégaze ce produit dans un dégazeur (5) dans lequel est insufflé un courant (6) d'air, d'azote ou d'oxygène (étape b); celui-ci élimine l'anhydre sulfureux (7) qui est recyclé vers le réacteur de photosulfonation (1).

Le produit de sulfonation ainsi dégazé (8) est introduit dans un mélangeur décanteur (9) dans lequel est également introduit une alcanolamine et/ou une alcoylamine (10). L'alcanolamine est choisie dans le groupe constitué par la mono-éthanolamine, la diéthanolamine, la triéthanol-amine et l'alcoylamine parmi la mono-isopropylamine, la diisopropylamine, la triisopropylamine ou leurs mélanges; ces produits sont en effet à la fois très polaires de sorte qu'ils sont immiscibles dans les esters gras et les paraffines de pétrole et, d'autre part, suffisamment basiques pour neutraliser les acides forts que sont les acides photosulfoniques.

Dans le cas des esters gras, les alcanolamines et/ou les alcoylamines ont le grand avantage de ne pas les saponifier aux températures voisines de l'ordinaire comme le ferait une solution de soude, base plus forte.

Pour extraire et neutraliser les acides photosulfoniques, il faut ajouter au mélange brut de photo-sulfonation plus d'alcanolamine et/ou d'alcoyl-amine qu'il n'en serait strictement nécessaire pour neutraliser les acides formés (le point de neutralisation exact étant facilement contrôlé en mesurant le pH correspondant qui est voisin de 6: on atteint ainsi un pH au moins égal à 8. On a observé aussi que d'élever la température au-delà de 50°C n'est pas nécessaire pour obtenir une bonne extraction, ce qui représente un avantage certain.

On obtient ainsi à l'issue de cette étape c, d'une part une phase légère dite raffinat (11) qui renferme les esters gras de méthyle ou les paraffines de pétrole insulfonés, et qui est recyclée vers le réacteur de photosulfonation (1) et, d'autre part, une phase lourde dite extrait (12) qui contient les acides sulfoniques ainsi que l'alcanolamine et la matière grasse insulfonée.

Cet extrait (12) est ensuite introduit dans un vaporisateur sous vide pour éliminer la matière grasse insulfonée et l'alcanolamine. Dans le cas des esters gras de méthyle, on peut aussi amidifier les insulfonés par l'alcanolamine résiduelle en utilisant les techniques bien connues de l'homme de l'art, ce qui évite, dans ce cas, l'étape de vaporisation.

Les exemples ci-dessous, qui n'ont aucun caractère limitatif, permettront de mieux comprendre comment la présente invention peut être mise en œuvre.

*Exemple I:*

On introduit 100 g de stéarate de méthyle dans le réacteur (2) où l'on réalise une photosulfonation dans les conditions habituelles, c'est-à-dire en y injectant un courant (3) gazeux d'anhydride sulfureux et d'oxygène et en l'irradiant simultanément par une lampe à mercure émettant dans l'ultraviolet. On obtient au bout d'une heure environ un produit (8) partiellement sulfoné pesant 106 g. On rajoute à ce produit (8) maintenu à 50°C 16 g de monoéthanolamine (10). Après agitation, le mélange décante rapidement et se sépare en deux parties: l'une dite raffinat (11) pesant 58 g, l'autre dite extrait (12) pesant 64 g. La composition du raffinat (11) et de l'extrait (12) est donnée dans le tableau I suivant, en poids pour cent.

*Tableau I*

| | Raffinat (58 g) | Extrait (64 g) |
|---|---|---|
| m SEA | < 1 | 60 |
| d SEA | 0 | 8 |
| SO$_4$ EA | 0 | 3 |
| MEA | 0,2 | 8 |
| FE | 98 | 21 |

m SEA   monosulfonate d'éthanolamine
d SEA   disulfonate d'éthanolamine
SO$_4$ EA  sulfate d'éthanolamine
MEA    monoéthanolamine
FE      ester gras, c'est-à-dire du stéarate de méthyle dans cet exemple

Les analyses ont été effectuées suivant les techniques bien connues de l'homme de l'art. L'acide monosulfonique, matière active, a été dosé par la méthode Epton. L'acide disulfonique a été dosé par une titrimétrie en milieu anhydre qui permet de doser, d'une part, la seconde acidité de l'acide sulfurique et, d'autre part, les acides forts constitués par la première acidité de l'acide sulfurique et l'acidité des acides sulfoniques. Les esters gras (stéarate de méthyle) ont été dosés par extraction à l'éther de pétrole. Enfin, la monoéthanolamine libre résiduelle a été dosée par titration à l'acide chlorhydrique.

On constate que le raffinat (11) ne contient pratiquement que des esters gras et qu'il peut par conséquent être directement recyclé vers l'étape de photosulfonation. L'extrait (12) contient tous les acides sulfoniques synthétisés, sous forme de sels de monoéthanolamine. On observe de plus que la photosulfonation ayant été réalisée à l'état anhydre, la quantité d'acide sulfurique formée n'est que de quelques pour-cent et par conséquent n'a pas à être éliminée du produit. Pour être utilisé comme matière active dans les lessives, l'extrait (12) n'a plus qu'à être débarrassé de la monoéthanolamine et des esters gras insulfonés résiduels qu'il contient, ce qui peut se faire facilement par évaporation sous un vide plus ou moins poussé.

*Exemple II:*

On introduit dans le réacteur de photosulfonation (2) 100 g d'une coupe paraffinique (1) comprenant 90% de n-alcanes ayant de 14 à 17 atomes de carbone, 5% maximum de n-alcanes ayant 12 à 13 atomes de carbone et 5% maximum de n-alcanes ayant 18 ou plus atomes de carbone. Le nombre de carbone moyen est de 15. Ce mélange est photosulfoné à l'état anhydre dans les conditions habituelles, c'est-à-dire en le faisant traverser par un courant (3) gazeux d'anhydride sulfureux et d'oxygène et en l'irradiant simultanément par une lampe à mercure émettant dans l'ultraviolet. On obtient au bout d'une heure environ un produit (8) partiellement sulfoné pesant 108 g. On rajoute au produit maintenu à 50°C 12 g de monoéthanolamine (10). Après agitation, le mélange décante rapidement et se sépare en deux parties: l'une dite raffinat (11) pèse 71 g, l'autre dite extrait (12) pèse 49 g. La composition du raffinat (11) et de l'extrait (12) est donnée dans le tableau II suivant, en poids pour cent.

*Tableau II*

|  | Raffinat (71 g) | Extrait (49 g) |
|---|---|---|
| m SEA | 0 | 62 |
| d SEA | 0 | 10 |
| SO$_4$ EA | 0 | 4 |
| MEA | 0,1 | 7 |
| FE | 99 | 17 |

On voit, comme dans le cas des esters gras, que le raffinat (11) à recycler ne contient que les paraffines insulfonées et que l'extrait (12) contenant les sulfonates recherchés peut être purifié facilement par évaporation de la monoéthanolamine et des matières grasses insulfonées.

Les produits obtenus selon l'invention sont particulièrement intéressants comme agents de surface, car ils ont des propriétés détergentes aussi bonnes que les sulfonates obtenus par sulfonation à l'anhydride sulfurique et sont de plus solubles dans l'eau à température ordinaire, du fait de la distribution du radical sulfonique le long de la chaîne, de sorte qu'il n'est pas besoin d'ajouter des agents hydrotopes aux lessives dont ils constituent la matière active.

## Revendications

1. Procédé pour préparer en continu des photosulfonates d'alcanolamine et/ou d'alcoylamine à partir d'un produit de départ (1) constitué par des esters gras de méthyle ou des paraffines de pétrole comprenant les étapes suivantes:

a) on soumet ledit produit de départ (1) à une photosulfonation en milieu anhydre par un mélange (3) d'anhydride sulfureux et d'oxygène sous irradiation ultra-violette;

b) on dégaze le produit (4) issu de l'étape a de l'anhydride sulfureux qu'il contient; procédé caractérisé par le fait qu'il comprend en outre les étapes suivantes:

c) on ajoute au produit dégazé (8) obtenu à l'issue de l'étape b une quantité d'alcanolamine et/ou d'alcoylamine (10) supérieure à celle qui est strictement nécessaire pour neutraliser les acides photosulfoniques contenus, de façon à décanter le mélange ainsi obtenu en deux phases, une phase légère dite raffinat (11) et une phase lourde dite extrait (12);

d) on recycle vers l'étape a ledit raffinat (11) qui renferme du produit de départ (1) insulfoné;

e) on recueille ledit extrait (12) qui renferme les photosulfonates d'alcanolamine et/ou d'alcoylamine.

2. Procédé selon la revendication 1, caractérisé par le fait que l'on ajoute à ladite étape c une quantité d'alcanolamine et/ou d'alcoylamine (10) suffisante pour élever le pH du mélange au moins jusqu'à 8.

3. Procédé selon l'une des revendications 1 ou 2, caractérisé par le fait que ladite étape c est effectuée à une température comprise entre 40 et 60° C.

4. Procédé selon l'une des revendications 1 à 3, caractérisé par le fait que ladite alcanolamine (10) utilisée est choisie dans le groupe constitué par la monoéthanolamine, la diéthanolamine, la triéthanolamine et/ou ladite alcoylamine parmi la mono-isopropylamine, la diisopropylamine et la triisopropylamine et leurs mélanges.

5. Procédé selon l'une des revendications 1 à 4, caractérisé par le fait que les esters gras de méthyle utilisés comme matière de départ sont obtenus par méthylation des huiles et des graisses naturelles d'origine animale ou végétale, suivie d'une extraction ou hydrogénation des composés insaturés.

6. Procédé selon l'une des revendications 1 à 4, caractérisé par le fait que les paraffines de pétrole utilisées comme matière de départ ont des chaînes carbonées droites comportant de 12 à 18 atomes de carbone, ou leur mélange.

## Patentansprüche

1. Verfahren zur kontinuierlichen Herstellung von Alkanolamin- und/oder Alkylamin-Photosulfonaten aus einem aus Fettsäuremethylestern oder aus Petroleumparaffinen bestehenden Ausgangsmaterial (1), wobei dieses Verfahren die folgenden Verfahrensstufen umfasst:

a) Man unterwirft dieses Ausgangsmaterial (1) in wasserfreiem Milieu einer Photosulfonierung durch ein Gemisch (3) aus Schwefelsäureanhydrid und Sauerstoff unter ultravioletter Strahlung;

b) man entgast das aus der Stufe a stammende Produkt (4) von dem darin enthaltenen Schwefelsäureanhydrid,

und wobei dieses Verfahren unter anderem die folgenden Verfahrensstufen umfasst:

c) man setzt dem aus der Stufe b stammenden, entgasten Produkt (8) eine solche Menge eines Alkanolamins und/oder Alkylamins (10) zu, die grösser ist als die Menge, welche erforderlich wäre, um die in diesem Produkt enthaltenen Photosulfonsäuren zu neutralisieren, unter solchen Bedingungen zu, dass sich das entstandene Gemisch in zwei Phasen trennt, nämlich eine als Raffinat (11) bezeichnete leichte Phase und eine als Extrakt (12) bezeichnete schwere Phase;

d) man führt dieses Raffinat (11), welches nicht-sulfoniertes Ausgangsmaterial (1) enthält, in die Stufe a zurück, und

e) man gewinnt diesen Extrakt (12), welcher die Alkanolamin- und/oder Alkylamin-Photosulfonate enthält.

2. Verfahren nach Anspruch 1, dadurch gekennzeichnet, dass man in dieser Stufe c eine solche Menge an Alkanolamin und/oder Alkylamin (10) zusetzt, die ausreicht, um den pH-Wert des Gemisches auf mindestens 8 zu erhöhen.

3. Verfahren nach Anspruch 1 oder 2, dadurch gekennzeichnet, dass diese Stufe c bei einer Temperatur zwischen 40 und 60° C durchgeführt wird.

4. Verfahren nach einem der Ansprüche 1 bis 3, dadurch gekennzeichnet, dass als dieses Alkanolamin Monoethanolamin, Diethanolamin und/oder Triethanolamin und dass als dieses Alkylamin Monoisopropylamin, Diisopropylamin und/oder Triisopropylamin, oder dass Gemische dieser Alkanolamine und/oder Alkylamine verwendet werden.

5. Verfahren nach einem der Ansprüche 1 bis 4, dadurch gekennzeichnet, dass die als Ausgangsmaterial verwendeten Fettsäuremethylester erhalten worden sind durch Methylierung von natürlichen pflanzlichen oder tierischen Ölen und Fetten mit anschliessender Extraktion oder Hydrierung der ungesättigten Verbindungen.

6. Verfahren nach einem der Ansprüche 1 bis 4, dadurch gekennzeichnet, dass die als Ausgangsmaterial verwendeten Petroleumparaffine solche mit geraden Kohlenstoffketten mit 12 bis 18 Kohlenstoffatomen oder dass sie Gemische solcher Paraffine sind.

## Claims

1. Process for the continuous preparation of alkanolamine and/or alkylamine photosulphonates from a starting material (1) constituted by fatty acid methyl esters or petroleum paraffins comprising the following steps:

a) submitting the said starting material (1) to photosulphonation under anhydrous conditions by a mixture (3) of sulphurous anhydride and oxygen under ultraviolet irradiation;

b) degassifying the product (4) from stage a of the sulphurous anhydride which it contains; the process being characterised in that it comprises in addition the following stages:

c) adding to the degassified product (8) obtained from stage b an amount of alkanolamine and/or alkylamine (10) greater than that strictly necessary for the neutralisation of the sulphonic acids contained in such a way as to separate the mixture thus obtained into two phases, a lighter phase called the raffinate (11) and a heavier phase called the extract (12);

d) recycling to stage a the said raffinate (11) which contains unsulphonated starting material (1);

e) recovering the said extract (12) which contains the alkanolamine and/or alkylamine polysulphonates.

2. Process according to Claim 1, characterised in that there is added in the said stage c an amount of alkanolamine and/or alkylamine (10) sufficient to raise the pH of the mixture to at least 8.

3. Process according to one of the Claims 1 or 2, characterised in that the said stage c is carried out at a temperature between 40 and 60°C.

4. Process according to any one of Claims 1 to 3, characterised in that the said alkanolamine (10) used is chosen from the group constituted by monoethanolamine, diethanolamine, triethanolamine and/or the said alkylamine amongst monoisopropylamine, diisopropylamine and triisopropylamine and their mixtures.

5. Process according to any one of Claims 1 to 4, characterised in that the fatty acid methyl esters used as starting material are obtained by methylation of natural oils and fats of animal or vegetable origin, followed by extraction or hydrogenation of the unsaturated compounds.

6. Process according to any one of Claims 1 to 4, characterised in that the petroleum paraffins used as starting material have straight carbon chains comprising 12 to 18 carbon atoms or their mixture.